# EUROPEAN PATENT SPECIFICATION

(11) **EP 1 355 639 B1**
(45) Date of publication and mention of the grant of the patent: **12.04.2006**
(21) Application number: 02716434.2
(22) Date of filing: 29.01.2002
(51) Int. Cl.: A61K 31/00, A61K 31/496, A61K 45/06, A61P 25/24, A61P 25/18, A61P 25/28, A61P 25/16, A61P 25/06, A61P 25/30, A61P 15/00, A61P 3/04, A61P 1/08, A61P 25/00, A61P 25/32

(54) **SUBSTITUTED CARBOSTYRIL DERIVATIVES AS 5-HT1A RECEPTOR SUBTYPE AGONISTS**
SUBSTITUIERTE CARBOSTYRILDERIVATE ALS 5-HT1A-SUBTYP AGONISTEN
DÉRIVÉS SUBSTITUÉS DE CARBOSTYRILE COMME AGONISTES DU SOUS-TYPE DU RECEPTEUR 5-HT 1A

(30) Priority: 29.01.2001 US 770210
(43) Date of publication of application: 29.10.2003
(60) Divisional application: 05023971.4
(73) Proprietor: OTSUKA PHARMACEUTICAL CO., LTD., Tokyo 101-8535 (JP)
(72) Inventor: JORDAN, Shaun, Maryland, MD 21704 (US); KIKUCHI, Tetsuro, Tokushima-shi, Tokushima 771-0104 (JP); TOTTORI, Katsura, Itano-gun, Tokushima 771-1345 (JP); HIROSE, Tsuyoshi, Tokushima-shi, Tokushima 770-0021 (JP); UWAHODO, Yasufumi, Tokushima-shi, Tokushima 771-1151 (JP)
(74) Representative: HOFFMANN EITLE
(86) International application number: PCT/JP2002/000626
(87) International publication number: WO 2002/060423

(56) References cited:
- EP-A- 0 367 141
- WO-A-92/20655
- WO-A-94/09765
- WO-A-94/13620
- WO-A-98/08817
- WO-A-99/38864
- US-A- 4 764 416
- US-A- 5 691 330
- LIEBERMAN JEFFREY A: "Atypical antipsychotic drugs as a first-line treatment of schizophrenia: A rationale and hypothesis." JOURNAL OF CLINICAL PSYCHIATRY, vol. 57, no. SUPPL. 11, 1996, pages 68-71, XP008005973 ISSN: 0160-6689
- DATABASE WPI Week 199806 Derwent Publications Ltd., London, GB; AN 1998-059088 XP002209299 JIYOUTEI YASUFUMI; NAKAI SHOZO ET AL.: "Antianxiety agent" & JP 09 301867 A (OTSUKA PHARMA CO LTD), 25 November 1997 (1997-11-25) cited in the application
- PRINSSEN ERIC P M ET AL: "Interactions between neuroleptics and 5-HT1A ligands in preclinical behavioral models for antipsychotic and extrapyramidal effects." PSYCHOPHARMACOLOGY, vol. 144, no. 1, May 1999 (1999-05), pages 20-29, XP002209298 ISSN: 0033-3158
- UWAHODO YASUFUMI ET AL: "Pharmacological profile of OPC-14597, a novel antipsychotic drug (2): Weak extrapyramidal side effects." JAPANESE JOURNAL OF PHARMACOLOGY, vol. 67, no. SUPPL. 1, 1995, page 144P XP008005977 68th Annual Meeting of the Japanese Pharmacological Society;Nagoya, Japan; March 25-28, 1995 ISSN: 0021-5198
- GARCIA-NANYA M., APIQUIAN R., FRESAN A.: "Atypical antipsychotics: Review article [Los antipsycoticos atipicos: Una revision]" SALUD MENTAL, vol. 24, no. 5, October 2001 (2001-10), pages 37-43, XP008005976
- KECK P E ET AL: "BIPOLAR DISORDER" MEDICAL CLINICS OF NORTH AMERICA, W. B. SAUNDERS COMPANY, PHILADELPHIA, US, vol. 3, no. 85, May 2001 (2001-05), pages 645-661, XP008005975 ISSN: 0025-7125
- JORDAN S ET AL: "IN VIVO EFFECTS OF ARIPIPRAZOLE ON DOPAMINERGIC AND SEROTONERGIC FUNCTION IN RAT PREFRONTAL CORTEX AND STRIATUM" SOCIETY FOR NEUROSCIENCE ABSTRACTS, SOCIETY FOR NEUROSCIENCE, US, vol. 2, no. 27, 2001, page 2327,AN87503 XP008005982 ISSN: 0190-5295
- AGNEW L.R.C. ET AL: 'Dorland's illustrated medical dictionary, 24th Edition', 1965, W.B. SAUNDERS COMPANY, PHILADELPHIA * page 1088 *
- BEERS M.H.; BERKOW R.: 'The Merck Manual of Diagnosis and Therapy, seventeenth edition', MERCK RESERACH LABORATORIES, WHITEHOUSE STATION, N.J. * page 1513 - page 1515 *
- ALFIERI ET AL: 'Comparative efficacy of a single oral dose of ondansetron and of buspirone against cisplatin-induced emesis in cancer patients' BRITISH JOURNAL OF CANCER vol. 72, 1995, pages 1013 - 1015, XP008046629
- MELTZER H.Y. ET AL: 'Single or multiple receptor targets: which are best for antipsychotic drugs' NEUROPSYCHOPHARMACOLOGY vol. 23, no. S2, 2000, page S73, XP008029267

## Description

### BACKGROUND OF THE INVENTION

### FIELD OF THE INVENTION

The present invention relates to the use of a carbostyril compound of the formula (1) or a salt thereof for the manufacture of a medicament for treating a patient suffering from a disorder of the central nervous system associated with the 5-HT_{1A} receptor subtype.

### RELATED ART

U.S. Patent No. 5,006,528; European Patent No. 367,141 and Japanese Patent Kokai (Laid-open)7-304,740 (1995) contain the same chemical structural formula as the carbostyril derivatives in the present invention, and their pharmacological properties are beneficial drug treatments for schizophrenia.

Carbostyril compounds, as well as those disclosed in Japanese Patent Kokai (Laid-open)9-301,867 (1997) are useful for the treatment of anxiety.

The carbostyril derivatives disclosed in European Patent No. 226,441 have the genus of the carbostyril derivatives in the present invention, and they are useful for the treatment of hypoxia.

In addition to the above, the carbostyril derivatives disclosed in U.S. Patent No. 4,734,416; Canadian Patent No. 1,117,110; British Patent No. 2,017,701; German Patent Nos. 2,911,108, 1,912,105 and 2,953,723; Japanese Patent Kokai(Laid-open)Nos. 54-130,587 (1979), 55-127, 371 (1980) and 62-149,664 (1987) have the genus of the carbostyril derivatives in the present invention, and they have antihistaminic activities and central nervous controlling activities.

It is reported that aripiprazole (7-{4-[4-(2,3-dichlorophenyl)-1-piperazinyl]butoxy}-3,4-dihydrocarbostyril, also known as, OPC-14597, BMS-337,039 and OPS-31) binds with high affinity to dopamine D₂ receptors and with moderate affinity to dopamine D₃ and 5-HT₇ receptors (Masashi Sasa et al., CNS Drug Reviews, Vol. 3, No. 1, pp. 24-33).

Further, it is reported that aripiprazole possesses presynaptic dopaminergic autoreceptor agonistic activity, postsynaptic D₂ receptor antagonistic activity, and D₂ receptor partial agonistic activity (T. Kikuchi, K. Tottori, Y. Uwahodo, T. Hirose, T. Miwa, Y. Oshiro and S. Morita: J. Pharmacol. Exp. Ther., Vol. 274, pp. 329, (1995); T. Inoue, M. Domae, K. Yamada and T. Furukawa: J. Pharmacol. Exp. Ther., Vol. 277, pp. 137, (1996)).

However, it has not been reported that compounds in the present invention have agonistic activity at 5-HT_{1A} receptor subtype.

It has been reported that therapeutic interventions using 5-HT_{1A} receptor ligands may be useful drug treatments for alcohol abuse (Mark Kleven et al., European Journal of Pharmacology, Vol. 281, (1995) pp. 219-228).

It is also reported that 5-HT_{1A} agonist drugs may be useful for the treatment and/or prophylaxis of disorders associated with neuronal degeneration resulting from ischemic events in mammals (U.S. Patent No. 5,162,375).

It is also reported that 5-HT_{1A} receptor hypersensitivity could be the biological basis for the increased frequency of migraine attack in stressful and anxious conditions (Massimo Leone et al., Neuro Report, Vol. 9, pp. 2605-2608(1998)).

It has recently been reported that (-)-(R)-2-[4-[[(3,4-dihydro-2H-1-benzopyran-2-yl)methyl]amino]-butyl]-1,2-benzisothiazol-3(2H)-one 1,1-dioxide monohydrochrolide (BAY-3702), a 5-HT_{1A} receptor agonist, has neuroprotective, anxiolytic- and antidepressant-like effects in animal models (Jean De Vry et al., European Journal of Pharmacology, Vol. 357, (1998), pp. 1-8).

It is also reported that 5-HT_{1A} receptor agonists appear to be broad spectrum antiemetic agents (Mary C. Wolff et al., European Journal of Pharmacology, Vol. 340, (1997), pp. 217-220; AB Alfieri et al., British Journal of Cancer, (1995), Vol. 72, pp. 1013-1015; Mary C. Wolff et al., Pharmacology Biochemistry and Behavior, 1995, Vol. 52, No. 3, pp. 571-575; James B. Lucot, European Journal of Pharmacology, 1997, Vol. 253, pp. 53-60).

Serotonin plays a role in several neurological and psychiatric disorders, including Alzheimer's disease, depression, nausea and vomiting, eating disorders, and migraine. (See Rasmussen et al., "Chapter 1. Recent Progress in Serotonin 5HT_{1A} Receptor Modulators", in Annual Reports in Medicinal Chemistry, Vol. 30, Section I, pp. 1-9, 1995, Academic Press, Inc.). WO 00/16777 discloses that a 5HT_{1A} receptor agonist, buspirone is efficacious in treating a variety of symptoms associated with ADHD, and that combined use of a D2 receptor agonist and 5-HT_{1A} agonist provides effective treatments for ADHD and Parkinson's disease.

5HT_{1A} agonists are effective in the treatment of cognitive impairment in Alzheimer's disease, Parkinson's disease or senile dementia. US 5824680 discloses that a 5-HT_{1A} agonist, ipsapirone, is effective in treating Alzheimer's disease by improving memory. US 4687772 describes that a 5-HT_{1A} partial agonist, buspirone, is useful for improving short term memory in patients in need of treatment. WO 93/04681 discloses that use of 5-HT_{1A} partial agonists have been used for the treatment or prevention of cognitive disorders associated with Alzheimer's disease, Parkinson's disease or senile dementia.

5HT_{1A} agonists are also effective in the treatment of depression. US 4771053 describes that a 5-HT_{1A} receptor partial agonist, gepirone, is useful in alleviation of certain primary depressive disorders, such as severe depression, endogenous depression, major depression with melancholia, and atypical depression. WO 01/52855 discloses that the combined use of the 5-HT_{1A} receptor partial agonist gepirone with an antidepressant can effectively treat depression.

The 5-HT_{1A} receptor partial agonist buspirone alleviates motor disorders such as neuroleptic induced parkinsonism and extrapyramidal symptoms. These observations are disclosed in US 4438119. Furthermore 5-HT_{1A} agonists reverse neuroleptic-induced catalepsy in rodents, which mimic movement impairments observed in Parkinson's disease (Mark J. Millan, Journal of Pharmacology and Experimental Therapeutics, 2000, Vol. 295, p853-861). Thus, aripiprazole can be used to manage psychosis in geriatric patients, Alzheimer's disease, Parkinson's disease or senile dementia, since it possesses potent, partial agonistic activities at D₂ and 5-HT_{1A} receptors. In addition, these patients might not experience extrapyramidal symptoms due to this property of aripiprazole.

Symptoms of patients corresponding to treatment-resistant and treatment-refractory schizophrenics involve not only the positive symptoms, but also the negative symptoms and emotional disorders, as well as cognitive impairments (i.e., cognitive dysfunction or cognitive disturbances) (K. Akiyama and S. Watanabe: Jpn. J. Clin. Psychopharmacol., Vol. 3, pp. 423, (2000)).

Cognitive impairment exists separately from the psychic symptoms in a schizophrenic individual. Thus, medical treatment is therefore quite important, because the cognitive impairment may disturb the socially adaptable behavior of these individuals (C. Hagger, P. Buckley, J. T. Kenny, L. Friedman, D. Ubogy and H. Y. Meltzer: Biol. Psychiatry, Vol. 34, pp. 702, (1993); T. Sharma and D. Mockler: J. Clin. Psychopharmacol., Vol. 18, (Suppl. 1), pp. 128, (1998)).

At present, clozapine is an antipsychotic drug that is effective against treatment-resistant schizophrenia. Clozapine (marketed under the name of Clozaril) was approved in 1990 by FDA for the treatment and management of severely ill schizophrenics who failed to respond adequately to standard antipsychotic therapy (M. W. Jann: Pharmacotherapy, Vol. 11, pp. 179, (1991)). Clozapine has been reported to be effective against cognitive impairments in treatment-resistant schizophrenics (C. Hagger, P. Buckley, J. T. Kenny, L. Friedman, D. Ubogy and H. Y. Meltzer: Biol. Psychiatry, Vol. 34, pp. 702, (1993); M. A. Lee, P. A. Thompson and H. Y. Meltzer: J. Clin. Psychiatry, Vol. 55 (Suppl. B), pp. 82, (1994); D. E. M. Fujii, I. Ahmed, M. Jokumsen and J. M. Compton: J. Neuropsychiatry Clin. Neurosci., Vol. 9, pp. 240, (1997)). For example, it is reported that clozapine improves cognitive impairments in attention, response time, fluent-speech, etc. in treatment-resistant schizophrenics (M. A. Lee, P. A. Thompson and H. Y. Meltzer: J. Clin. Psychiatry, Vol. 55 (Suppl. B), pp. 82, (1994)). It has been also reported that clozapine provides effective improvements in cognitive impairments in an objective evaluation scale of the Wechsler Adult Intelligence Scale-Revised Full Scale (D. E. M. Fujii, I. Ahmed, M. Jokumsen and J. M. Compton: J. Neuropsychiatry Clin. Neurosci., Vol. 9, pp. 240, (1997)).

The 5-HT_{1A} receptor has been demonstrated to play a role in the therapeutic efficacy of clozapine against cognitive impairments. This relationship was revealed by a binding experiment using human the 5-HT_{1A} receptors (S. L. Mason and G. P. Reynolds: Eur. J. Pharmacol., Vol. 221, pp. 397, (1992)). Further, in accordance with progress in molecular pharmacology, it is clearly understood that 5-HT_{1A} receptor agonistic activity or 5-HT_{1A} receptor partial agonistic activity plays an important role in cognitive impairments (A. Newman-Tancredi, C. Chaput, L. Verriele and M. J. Millan: Neuropharmacology, Vol. 35, pp. 119, (1996)). Additionally, it was reported that the number of 5-HT_{1A} receptor is increased in the prefrontal cortex of chronic schizophrenics who were classified treatment-resistant. This observation was explained by a compensatory process where by the manifestation of severe symptoms of chronic schizophrenia are a result of impaired neuronal function mediated by hypofunctional 5-HT_{1A} receptors (T. Hashimoto, N. Kitamura, Y. Kajimoto, Y. Shirai, O. Shirakawa, T. Mita, N. Nishino and C. Tanaka: Psychopharmacology, Vol. 112, pp. S35, (1993)). Therefore, a lowering in neuronal transmission mediated through 5-HT_{1A} receptors is expected in treatment-resistant schizophrenics. Thus the clinical efficacy of clozapine may be related to its partial agonist efficacy at the 5-HT_{1A} receptors (A. Newman-Tancredi, C. Chaput, L. Verriele and M. J. Millan: Neuropharmacology, Vol. 35, pp. 119, (1996)). 5-HT_{1A} receptor agonistic activity may be related to the clinical effects of clozapine, and this hypothesis is supported by a positron emission tomography study in primates which showed that clozapine interacts with brain 5-HT_{1A} receptors at a therapeutically effective dose (Y. H. Chou, C. Halldin and L. Farde: Int. J. Neuropsychopharmacol., Vol. 4 (Suppl. 3), pp. S130, (2000)). Furthermore tandospirone, which is known as a selective 5-HT_{1A} receptor agonist, improved cognitive impairments in chronic schizophrenic patients (T. Sumiyoshi, M. Matsui, I. Yamashita, S. Nohara, T. Uehara, M. Kurachi and H. Y. Meltzer: J. Clin. Pharmacol., Vol. 20, pp. 386, (2000)). While, in animal tests, all reports do not always suggest that 5-HT_{1A} receptor agonist activity may be related to cognitive impairment, however, 8-OH-DPAT (8-hydroxy-2-(di-n-propylamino)tetralin), which is known as a selective 5-HT_{1A} receptor agonist, improves learning and memory impairments induced by scopolamine known as a muscarinic receptor antagonist, suggesting a relationship between 5-HT_{1A} receptor agonistic activity and improvements in cognitive impairments (M. Carli, P. Bonalumi, R. Samanin: Eur. J. Neurosci., Vol. 10, pp. 221, (1998); A. Meneses and E. Hong: Neurobiol. Learn. Mem., Vol. 71, pp. 207, (1999)).

Atypical antipsychotic drugs, such as risperidone and olanzapine, were marketed after clozapine, and it is reported that these drugs improve cognitive impairments in treatment-resistant schizophrenics (M. F. Green, B. D. Marshall, Jr., W. C. Wirshing, D. Ames, S. R. Marder, S. McGurck, R. S. Kern and J. Mintz: Am. J. Psychiatry, Vol. 154, pp. 799, (1997); G. Bondolifi, H. Dufour, M. Patris, J. P. May, U. Billeter, C. B. Eap and P. Baumann, on behalf of the risperidone Study Group: Am. J. Psychiatry, Vol. 155, pp. 499, (1998); A. Breier, S. H. Hamilton: Biol. Psychiatry, Vol. 45, pp. 403, (1999)).

As explained above, 5-HT_{1A} receptor agonistic activity is important for improving cognitive impairment caused by treatment-resistant schizophrenia. Clozapine is effective against treatment-resistant schizophrenia, however, its use is limited due to its severe side-effect of producing agranulocytosis which requires patients to undergo periodical blood tests. Under these circumstances, the development of a safe anti-psychotic drug with potent, full or partial agonist activity at 5-HT_{1A} receptors is earnestly desired.

The carbostyril compound in the present invention binds with high affinity and displays a potent, partial agonist activity at the 5-HT_{1A} receptors and it has higher intrinsic activity (about 68%) as compared with that of clozapine. Therefore, the compound in the present invention has a 5-HT_{1A} receptor agonistic activity that is more potent than the agonistic activity of clozapine. Thus, the present carbostyril compound may represent a more potent and highly safe drug for curing cognitive impairments caused by treatment-resistant schizophrenia, inveterate schizophrenia, chronic schizophrenia, and the like, as compared with other currently available pharmacotherapeutic treatments. That is, the compound in the present invention may prove to be a potent and safer drug therapy for cognitive impairments caused by treatment-resistant schizophrenia, inveterate schizophrenia, chronic schizophrenia, etc., which fail to respond adequately to currently available antipsychotic drugs such as chlorpromazine, haloperidol, sulpiride, fluphenazine, perphenazine, thioridazine, pimozide, zotepine, risperidone, olanzapine, quetiapine, amisulpride, etc.

In particular, the carbostyril compound in the present invention may be a potent and highly safe drug therapy against cognitive impairments caused by treatment-resistant schizophrenia, inveterate schizophrenia, chronic schizophrenia, etc. which fail to respond adequately to both of 1 to 3 typical antipsychotic drugs selected from the group consisting of chlorpromazine, haloperidol and perphenazine, and one atypical antipsychotic drug selected from risperidone, olanzapine, quetiapine and amisulpride.

Moreover, the compound in the present invention may be a potent and highly safe drug therapy against cognitive impairments caused by treatment-resistant schizophrenia, inveterate schizophrenia, chronic schizophrenia, etc. which fail to respond adequately to both of 2 typical antipsychotic drugs selected from chlorpromazine, haloperidol and perphenazine, and one atypical antipsychotic drug selected from risperidone, olanzapine, quetiapine and amisulpride.

Moreover, the compound in the present invention may be a potent and highly safe drug therapy against cognitive impairments caused by treatment-resistant schizophrenia, inveterate schizophrenia, chronic schizophrenia, etc. which fail to respond adequately to both of 1 to 2 typical antipsychotic drugs selected from chlorpromazine and haloperidol, and one atypical antipsychotic drug selected from risperidone, olanzapine, quetiapine and amisulpride.

Moreover, the compound in the present invention may be a potent and highly safe drug therapy against cognitive impairments caused by treatment-resistant schizophrenia, inveterate schizophrenia, chronic schizophrenia, etc. which fail to respond adequately to both of 2 typical antipsychotic drugs selected from chlorpromazine and haloperidol, and one atypical antipsychotic drug selected from risperidone, olanzapine, quetiapine and amisulpride.

J.A. Lieberman, J. Clin. Psychiatry 1996, 57 [suppl. 11]: 68-71, reports on atypical antipsychotic drugs for the treatment of schizophrenia. Among the compounds mentioned, aripiprazole, which is one of the compounds of the present claims, is referred to as OPC-14597 in Table 3 on page 96. The compounds are considered to be putative atypical antipsychotic drugs with, as compared with conventional antipsychotic drugs, novel pharmacological profiles. Their potential use for treating schizophrenia is described.

US 4,734,416 concerns carbostyril derivatives having antihistaminic and central nervous controlling actions that can be used for the treatment of various diseases, including anxiety and manic depressive psychosis and as sleep, inducing agents. The general formula shown in e.g. column 1, lines 50-60, generically includes the compounds of the present formula (1).

JP-A-9301867 discloses the utility of 7-{4-[4-(2,3-dichlorophenyl)-1-piperzinyl]butoxy}-3,4-dihydrocarbostyril and salts thereof as an anxiolytic agent. These compounds are included within formula (1) of the present invention.

WO 94/09765 is directed to the use of a cortical growth factor S-100_{B} as trophic for cortical and serotonergic neurons in the brain as well as the use of 5-HT--_{1A} agonists, S-100_{B} and derivatives thereof to induce cortical or serotonergic growth, stimulation or regeneration for therapeutic and/or diagnostic applications. The diseases addressed in this document comprise, among others, autism, depression, Down's syndrome and Alzheimer's disease biological rhythm-based sleep disorder as well as alcohol and drug abuse.

WO 99/38864 is directed to oxazole derivatives, which are useful for the treatment of conditions related to or affected by the 5-HT_{1A} receptor subtype. The compounds are described as being particularly useful for the treatment of psychosis, e.g. schizophrenia, anxiety, depression and related CNS disorders and other conditions such as the treatment of alcohol and drug withdrawal, sexual dysfunction and memory deficits associated with Alzheimer's disease.

WO 92/20655 describes specific carboxamino-(1,2N)-carbocyclic-2-amino-1,2,3,4-tetrahydro-2-naphthylen-derivatives useful as drugs for the treatment of psychiatric diseases that are thought to be due to dysfunctions in monoaminergic neuronal systems, particularly those involving serotonin (5-HT)- and dopamine (DA), and especially of diseases associated with 5-HT_{1A} receptor subtypes. Diseases to be treated with the compounds in this document include depression, OCD, migraine, sexual dysfunction, schizophrenia, Parkinson's disease, and others.

H.Y. Meltzer et al., Neuropsychopharmacology 2000, Vol. 23, Nr. S2, S73, concerns single or multiple receptor targets for antipsychotic drugs. It is described that a variety of second generation atypical pharmaceutical drugs, including aripiprazole, are multi-receptor antagonists, which show, among others, a 5-HT_{1A} partial agonism, and may be useful in the treatment of cognitive impairment.

### SUMMARY OF THE INVENTION

It is an object of the present invention to provide a medicament for treating a patient suffering from a disorder of the central nervous system associated with the 5-HT_{1A} receptor subtype.

Thus, the present invention is directed to the use of a carbostyril compound of the formula (1): 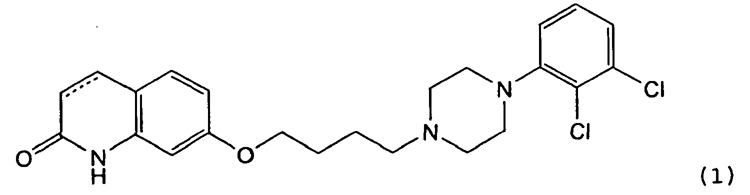 wherein the dotted line represents a single or a double bond, or a pharmaceutically acceptable salt or solvate thereof, for the production of a medicament effective in the treatment of disorders of the central nervous system associated with 5-HT_{1A} receptor subtype, selected from depression; cognitive impairment; autism; Down's syndrome; attention deficit hyperactivity disorder (ADHD); neurodegenerative diseases selected from Alzheimer's disease and Parkinson's disease; obsessive compulsive disorder (OCD); sleep disorders; sexual dysfunction; alcohol abuse; drug addiction; emesis; motion sickness; obesity; and migraine.

### DETAILED DESCRIPTION OF THE INVENTION

As the 5-HT_{1A} receptor subtype agonist compound for use in accordance with the present invention carbostyril derivatives represented by the following formula (1) are used: 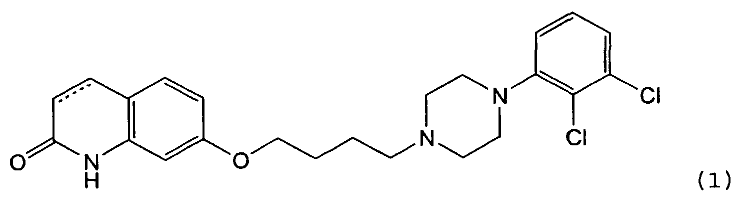 wherein the dotted line represents a single or a double bond.

The compounds of the foregoing general formula (1) are known compounds, which are disclosed in publications such as US 5,006,528 or which can be readily prepared by the processes described in the above publication.

The carbostyril derivative represented by the formula (1) in the present invention can easily be converted into its acid-addition salt by reacting it with a pharmaceutically acceptable acid. Examples of such acid include inorganic acids, such as hydrochloric acid, sulfuric acid, phosphoric acid, hydrobromic acid and the like; organic acids, such as oxalic acid, maleic acid, fumaric acid, malic acid, tartaric acid, citric acid, benzoic acid and the like.

The solvent of solvates is a solvent conventionally used in recrystallization. Examples of solvates include hemihydrates, hydrates, and alcoholates, such as ethanolates, methanolates and isopropanolates.

The desired compounds, prepared by the reactions mentioned above, can easily be isolated and purified by usual separation procedures such as solvent extraction, dilution, recrystallization, column chromatography, preparative thin layer chromatography and the like.

The potent, partial 5-HT_{1A} receptor agonist in the present invention is useful for various disorders of the central nervous system associated with the 5-HT_{1A} receptor subtype that induces depression, such as endogenous depression, major depression, melancholia, and treatment-resistant depression; obsessive compulsive disorder (OCD); sleep disorders; sexual dysfunction; alcohol abuse and drug addiction; cognitive impairment; neurodegenerative diseases selected from Alzheimer's disease and Parkinson's disease, cognitive impairments caused by neurodegenerative diseases such as Alzheimer's disease and Parkinson's disease; emesis; motion sickness; obesity; migraine; autism; Down's syndrome; attention-deficit hyper-activity disorder (ADHD) and cognitive impairments caused by treatment-resistant schizophrenia, inveterate schizophrenia or chronic schizophrenia.

Compounds of the present invention may be suitably prepared into pharmaceutically acceptable formulations (see U.S. Patent No. 5,006,528, European Patent No. 367,141 and Japanese Kokai (Laid-open) 7-304,740 (1995), and Japanese Patent Application No. 2000-194976).

The dosage of these pharmaceutical preparations of the invention may be selected appropriately depending on the method of administration, the patient's age, sex and other factors, severity of the disease and other factors. Generally, however, the daily dose of the active ingredient compound is preferably within the range of 0.0001 to 50 mg per kilogram of body weight. It is desirable that the active ingredient compound be contained in each unit dosage form in an amount of 0.001 to 1,000 mg, particularly 0.01 to 100 mg, more particularly 0.1 to 50 mg, yet more particularly 1 mg to 20 mg.

### Pharmacological tests

### 1. MATERIALS AND METHODS

### 1.1 Test Compound

7-{4-[4-(2,3-Dichlorophenyl)-1-piperazinyl]-butoxy}-3,4-dihydrocarbostyril (aripiprazole) was used as test compound.

### 1.2 Reference Compounds

Serotonin (5-HT) and WAY-100635 (N-[2-[4-(2-methoxyphenyl)-1-piperazinyl]ethyl]-N-(2-pyridimyl)-cyclohexanecarboxamide, a 5-HT_{1A} receptor antagonist, manufactured by RBI (Natick, MA) were used as reference compounds.

### 1.3 Vehicle

Dimethyl sulfoxide (DMSO) manufactured by Sigma Chemical Co. (St. Louis, MO) was used as vehicle.

### 1.4 Preparation of Test and Reference Compounds

Test compound was dissolved in 100% dimethyl sulfoxide (DMSO) to yield 100 µM stock solutions (final concentration of DMSO in all tubes containing test compound was 1%, v/v). All other reference compounds were prepared by the same method using double-distilled water rather than DMSO.

### 1.5 Experimental Procedure for the [³⁵S]GTP_{γ}S Binding Assay

Test and reference compounds were studied in triplicate at 10 different concentrations (0.01, 0.1, 1, 5, 10, 50, 100, 1000, 10000 and 50000 nM) for their effects upon basal [³⁵S]GTP_{γ}S binding to h5-HT_{1A} CHO cell membranes. Reactions were performed in 5 ml glass test tubes containing 8 µl of test/reference drug mixed with 792 µl of buffer (25 mM Tris HCl, 50 mM NaCl, 5 mM MgCl₂, 0.1 mM EGTA, pH = 7.4) containing GDP (1 µM), [³⁵S]GTPγS (0.1 nM) and h5-HT_{1A} CHO cell membranes (10 µg protein/reaction; NEN Life Science Products, Boston, MA; catalog # CRM035, lot # 501-60024, GenBank # X13556). Reactions proceeded for 60 min at room temperature and were terminated by rapid filtration through Whatman GF/B filter paper, using a Brandel harvester and 4x3 ml ice-cold buffer washes. ³⁵S radioactivity bound to the filter paper was measured using liquid scintillation counting (1272 Clinigamma, LKB/Wallach).

### 1.6 Experimental Procedure to Determine the Binding Affinity of Test compound (aripiprazole) at the h5-HT_{1A} Receptor

Test compound was studied in triplicate at 10 different concentrations (0.01, 0.1, 1, 10, 50, 100, 500, 1000, 5000 and 10000 nM) to determine its displacement of [³H]8-OH-DPAT (1 nM; NEN Life Sciences; catalog # NET 929, lot # 3406035, Specific Activity = 124.9 Ci/mmol) binding to h5-HT_{1A} receptors in CHO cell membranes (15 - 20 µg protein; NEN Life Science Products, catalog # CRM035, lot # 501-60024). Membranes (396 µl) were incubated in 5 ml glass tubes containing [³H]8-OH-DPAT (396 µl), test compound or vehicle (8 µl) and buffer A (50 mM Tris.HCl, 10 mM MgSO₄, 0.5 mM EDTA, 0.1% (w/v) ascorbic acid, pH = 7.4). All assays proceeded for 60 min at room temperature and were terminated by rapid filtration through Whatman GF/B filter paper (presoaked in buffer B; 50 mM Tris.HCl, pH = 7.4), using a Brandel harvester and 4×1 ml ice-cold washes with buffer B. Non-specific binding was determined in the presence of 10 µM (+)8-OH-DPAT.

### 1.7 Parameters Determined

Serotonin (5-HT) is a full 5-HT_{1A} receptor agonist which stimulates increases in basal [³⁵S]GTP_{γ}S binding to h5-HT_{1A} receptors in recombinant CHO cell membranes. Test compound was studied at 10 concentrations to determine their effects upon basal [³⁵S]GTPγS binding relative to that produced by 10 µM 5-HT. The relative potency (EC₅₀, 95% confidence interval) and intrinsic agonist activity (% of Eₘₐₓ for 10 µM 5-HT) was calculated for each compound by computerized non-linear regression analysis of complete concentration-effect data. The binding affinity of test compound at the h5-HT_{1A} receptor was determined by its ability to prevent [³H]8-OH-DPAT binding to CHO cell membranes that express this receptor. Non-linear regression analysis of the competition binding data was used to calculate an inhibition constant (IC₅₀, 95% confidence interval), which is the concentration of test compound that occupies half of the h5-HT_{1A} sites specifically bound by [³H]8-OH-DPAT. The affinity of h5-HT_{1A} receptors for test compound (Ki, 95% confidence interval) was calculated by the equation, Ki = (IC₅₀)/(1+([[³H]8-OH-DPAT]/Kd), where the Kd for [³H]8-OH-DPAT at h5-HT_{1A} = 0.69 nM (NEN Life Sciences). All estimates of drug binding affinity, potency and intrinsic efficacy at the h5-HT_{1A} receptor were calculated using GraphPad Prism version 3.00 for Windows (GraphPad Software, San Diego, CA).

### 2. RESULTS

Test compound and 5-HT produced concentration-dependent increases above basal [³⁵S]GTPγS binding. 1% DMSO tested alone had no effect upon basal or drug-induced [³⁵S]GTP_{γ}S binding.

Test compound (EC₅₀ = 2.12 nM), 5-HT (EC₅₀ = 3.67 nM), potently stimulated basal [³⁵S]GTP_{γ}S binding. Potency and intrinsic agonist efficacy estimates were derived by non-linear regression analysis with correlation coefficients (r²)>0.98 in each case (Table 1). Test compound exerted partial agonist efficacies in the 65 - 70% range. WAY-100635 produced no significant change (unpaired Student's t-test) in basal [³⁵S]GTPγS binding at all concentrations tested (Table 1). WAY-100635 did, however, completely inhibit the effects of 5-HT and test compound upon [³⁵S]GTP_{γ}S binding to h5-HT_{1A} receptors in CHO cell membranes (Table 2). Tables 1 and 2 are shown below.

Test compound demonstrated high affinity binding to h5-HT_{1A} receptors in CHO cell membranes (IC₅₀ = 4.03 nM, 95% confidence interval = 2.67 to 6.08 nM; Ki = 1.65 nM, 95% confidence interval = 1.09 to 2.48 nM).

**Table 1 Potency (EC₅₀) and Intrinsic Agonist Efficacy (Eₘₐₓ) of Test compound and Reference Drugs in a h5-HT_{1A} [³⁵S]GTP_{γ}S CHO-cell Membrane Binding Assay.**

| Drug | EC₅₀, nM (95% Confidence Interval) | Eₘₐₓ (% ± SEM) | Goodness of Fit (r²) |
|---|---|---|---|
| Test Compound | 2.12 (0.87 to 5.16) | 68.13 ± 3.16 | 0.986 |
| 5-HT | 3.67 (1.56 to 8.63) | 98.35 ± 4.47 | 0.986 |
| WAY-100635 | - | - | - |

**Table 2 Inhibitory Potency (IC₅₀) of WAY-100635 versus 1 µM Concentration of 5-HT and Test compound in a h5-HT_{1A} [³⁵S]GTP_{γ}S CHO-cell Membrane Binding Assay.**

| Drug Combination | WAY-100635 Inhibition Potency, IC₅₀, nM (95% Confidence Interval) | Goodness of Fit (r²) |
|---|---|---|
| 5-HT + WAY-100635 | 217.1 (127.4 to 369.7) | 0.988 |
| Test compound + WAY-100635 | 392.2 (224.1 to 686.2) | 0.989 |

## Claims

1. Use of a carbostyril compound of the formula (1): 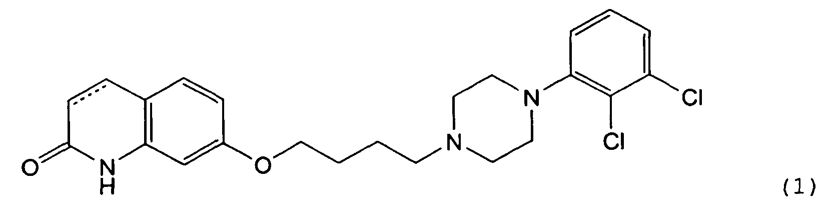 wherein the dotted line represents a single or a double bond, or a pharmaceutically acceptable salt or solvate thereof, for the production of a medicament effective in the treatment of disorders of the central nervous system associated with 5-HT_{1A} receptor subtype, selected from depression; cognitive impairment; autism; Down's syndrome; attention deficit hyperactivity disorder (ADHD); neurodegenerative diseases selected from Alzheimer's disease and Parkinson's disease; obsessive compulsive disorder (OCD); sleep disorders; sexual dysfunction; alcohol abuse; drug addiction; emesis; motion sickness; obesity; and migraine.

2. The use of Claim 1 wherein the disorder is depression.

3. The use of Claim 2 wherein the depression is selected from endogenous depression, major depression, melancholia and treatment-resistant depression.

4. The use of claim 1 wherein the disorder is cognitive impairment.

5. The use of claim 4 wherein the cognitive impairment is caused by Alzheimer's disease or Parkinson's disease.

6. The use of claim 1 wherein the disorder is autism, Down's syndrome or attention deficit hyperactivity disorder (ADHD).

7. The use of claim 1 wherein the disease is a neurodegenerative disease selected from Alzheimer's disease and Parkinson's disease.

8. The use of claim 1 wherein the disorder is obsessive compulsive disorder (OCD); sleep disorders; sexual dysfunction; alcohol abuse; drug addiction; emesis; motion sickness; obesity; or migraine.

9. The use of any of the preceding claims wherein the carbostyril compound is 7-{4-[4-(2,3-dichlorophenyl)-1-piperazinyl]butoxy}-3,4-dihydrocarbostyril.

## Patentansprüche

1. Verwendung einer Carbostyrylverbindung der Formel (1) : 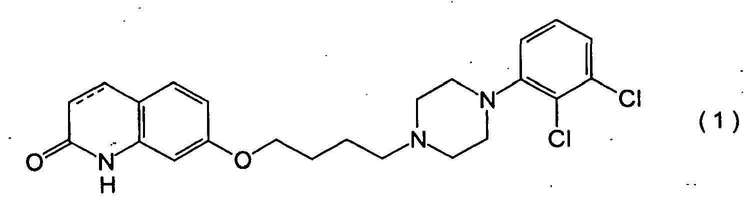 worin die gepunktete Linie eine Einfach- oder Doppelbindung ist, oder eines pharmazeutisch annehmbaren Salzes oder Solvats davon zur Herstellung eines Medikaments, das wirksam ist zur Behandlung von Erkrankungen des Zentralnervensystems, die mit einem 5-HT_{1A}-Rezeptor-Subtypen in Verbindung stehen, ausgewählt aus Depression; Wahrnehmungsbeeinträchtigung; Autismus; Down-Syndrom; aufmerksamkeitsdefizitäre Hyperaktivitätserkrankung (ADHD); neurodegenerativen Erkrankungen, ausgewählt aus Alzheimer und Parkinson; Zwangsneurose (OCD); Schlafstörungen; sexueller Dysfunktion; Alkoholmissbrauch; Drogenabhängigkeit; Erbrechen; Reiseübelkeit; Fettleibigkeit und Migräne.

2. Verwendung gemäss Anspruch 1, worin die Erkrankung Depression ist.

3. Verwendung gemäss Anspruch 2, worin die Depression ausgewählt ist aus endogener Depression, grosser Depression, Melancholie und behandlungsresistenter Depression.

4. Verwendung gemäss Anspruch 1, worin die Erkrankung Wahrnehmungsbeeinträchtigung ist.

5. Verwendung gemäss Anspruch 4, worin die Wahrnehmungsbeeinträchtigung durch Alzheimer oder Parkinson hervorgerufen wird.

6. Verwendung gemäss Anspruch 1, worin die Erkrankung Autismus, Down-Syndrom oder aufmerksamkeitsdefizitäre Hyperaktivitätserkrankung (ADHD) ist.

7. Verwendung gemäss Anspruch 1, worin die Erkrankung eine neurodegenerative Erkrankung ist, ausgewählt aus Alzheimer und Parkinson.

8. Verwendung gemäss Anspruch 1, worin die Erkrankung Zwangsneurose, Schlafstörungen, sexuelle Dysfunktion, Alkoholmissbrauch, Drogenabhängigkeit, Erbrechen, Reiseübelkeit; Fettleibigkeit oder Migräne ist.

9. Verwendung gemäss mindestens einem der vorhergehenden Ansprüche, worin die Carbostyrylverbindung 7-{4-[4-(2,3-Dichlorphenyl)-1-piperazinyl]butoxy}-3,4-dihydrocarbostyryl ist.

## Revendications

1. Utilisation d'un composé carbostyrile de formule (1) : 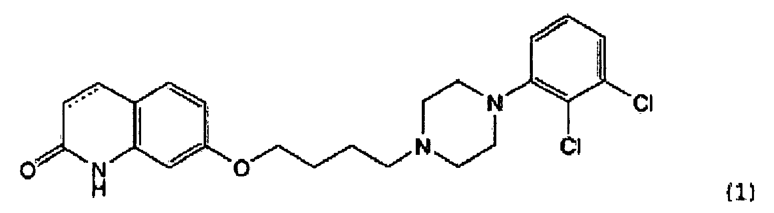 dans lequel la ligne pointillée représente une simple ou une double liaison, ou l'un de ses sels ou solvats pharmaceutiquement acceptables, pour la production d'un médicament efficace dans le traitement de troubles du système nerveux central associés au sous-type de récepteur 5-HT_{1A}, choisis parmi une dépression ; un trouble cognitif ; l'autisme ; le syndrome de Down ; un trouble déficitaire de l'attention avec hyperactivité (TDHA) ; des maladies neurodégénératives choisies parmi la maladie d'Alzheimer et la maladie de Parkinson ; un trouble obsessionnel compulsif (TOC) ; des troubles du sommeil ; un dysfonctionnement sexuel ; l'alcoolisme ; une dépendance aux drogues ; des vomissements ; le mal des transports ; l'obésité ; et la migraine.

2. Utilisation de la revendication 1, dans laquelle le trouble est une dépression.

3. Utilisation de la revendication 2, dans laquelle la dépression est choisie parmi une dépression endogène, une dépression majeure, une mélancolie et une dépression résistant aux traitements.

4. Utilisation de la revendication 1, dans laquelle le trouble est un trouble cognitif.

5. Utilisation de la revendication 4, dans laquelle le trouble cognitif est provoqué par la maladie d'Alzheimer ou la maladie de Parkinson.

6. Utilisation de la revendication 1, dans laquelle le trouble est l'autisme, le syndrome de Down ou un trouble déficitaire de l'attention avec hyperactivité (TDHA).

7. Utilisation de la revendication 1, dans laquelle la maladie est une maladie neurodégénérative choisie parmi la maladie d'Alzheimer et la maladie de Parkinson.

8. Utilisation de la revendication 1, dans laquelle le trouble est un trouble obsessionnel compulsif (TOC) ; des troubles du sommeil ; un dysfonctionnement sexuel ; l'alcoolisme ; une dépendance aux drogues ; des vomissements ; le mal des transports ; l'obésité ; ou la migraine.

9. Utilisation de l'une quelconque des revendications précédentes dans laquelle le composé carbostyrile est le 7-{4-[4-(2,3-dichlorophényl)-1-pipérazinyl]butoxy}-3,4-dihydrocarbostyrile.
